(19) Europäisches Patentamt / European Patent Office / Office européen des brevets

(11) **EP 1 085 467 B1**

(12) **EUROPÄISCHE PATENTSCHRIFT**

(45) Veröffentlichungstag und Bekanntmachung des Hinweises auf die Patenterteilung:
**07.03.2007 Patentblatt 2007/10**

(51) Int Cl.:
***G06T 11/00*** *(2006.01)*

(21) Anmeldenummer: **00203160.7**

(22) Anmeldetag: **12.09.2000**

(54) **Computertomographie-Verfahren mit helixförmiger Relativbewegung**

Computer tomography method with helicoidal scanning

Procédé de tomographie par ordinateur avec balayage hélicoidal

(84) Benannte Vertragsstaaten:
**DE FR GB NL**

(30) Priorität: **18.09.1999 DE 19944701**

(43) Veröffentlichungstag der Anmeldung:
**21.03.2001 Patentblatt 2001/12**

(73) Patentinhaber:
- **Philips Intellectual Property & Standards GmbH
20099 Hamburg (DE)**
Benannte Vertragsstaaten:
**DE**
- **Koninklijke Philips Electronics N.V.
5621 BA Eindhoven (NL)**
Benannte Vertragsstaaten:
**FR GB NL**

(72) Erfinder:
- **Danielsson, Per-Erik
52064 Aachen (DE)**
- **Turbell, Henrik
52064 Aachen (DE)**

(74) Vertreter: **Volmer, Georg et al
Philips Intellectual Property & Standards GmbH,
Postfach 50 04 42
52088 Aachen (DE)**

(56) Entgegenhaltungen:
| | |
|---|---|
| **EP-A- 1 077 429** | **EP-A- 1 077 430** |
| **WO-A-99/36885** | **US-A- 5 881 122** |

- **TURBELL H., DANIELSSON P.E.: "An improved PI-method for reconstruction from helical cone-beam projections" NUCLEAR SCIENCE SYMPOSIUM / MEDICAL IMAGING CONFERENCE, [Online] 28. Oktober 1999 (1999-10-28), Seiten 865-868 im Conf. Record, Volume 2, XP002192546 Seattle, USA Gefunden im Internet: <URL:http://ieeexplore.ieee.org, http://wwwrad.med.utah.edu/mirl/mic99/abst racts.html> [gefunden am 2002-03-06]**
- **P.E. DANIELSSON, P. EDHOLM, J. ERIKSSON, M. MAGNUSSON SEGER: "Towards Exact 3D-Reconstruction for Helical Cone-Beam Scanning of Long Objects. A New Detector Arrangement and a New Completeness Condition - Poster" INT. MEETING ON FULLY THREE-DIMENSIONAL IMAGE RECONSTRUCTION IN RADIOLOGY AND NUCLEAR MEDICINE, 27. Juni 1997 (1997-06-27), XP002192547 Pittsburgh PA, USA**

EP 1 085 467 B1

**Beschreibung**

Die Erfindung betrifft ein Computertomographie-Verfahren mit den Schritten

- Erzeugen eines kegelförmigen, einen Untersuchungsbereich bzw. ein darin befindliches Objekt durchsetzenden Strahlenbündels mit einer Strahlenquelle,
- Erzeugung einer eine Rotation um eine Rotationsachse und eine Verschiebung parallel zur Rotationsachse umfassenden Relativbewegung zwischen der Strahlenquelle einerseits und dem Untersuchungsbereich bzw. dem Objekt andrerseits in Form einer Helix,
- Akquisition von Meßwerten, die von der Intensität in dem Strahlenbündel jenseits des Untersuchungsbereiches abhängen, mit einer Detektoreinheit während der Relativbewegung,
- Rebinning der Meßwerte zu einer Anzahl von Gruppen von Meßdaten,
- Filterung der durch das Rebinning erzeugten Meßdaten der Gruppen, die Filteroperationen an verschiedenen Untergruppen von Meßdaten umfaßt,
- Rekonstruktion der Absorption in Voxeln des Untersuchungsbereiches durch Rückprojektion der gefilterten Meßdaten von mehreren Gruppen.

Ein solches Computertomographie-Verfahren ist aus der der WO 99/36885 bekannt. Das Rebinning liefert Gruppen von Meßdaten, die zu Strahlen gehören, die sich ergeben würden, wenn ein ebener, rechteckiger Detektor in einer die Rotationsachse enthaltenen Ebene die Meßdaten einer sich entlang eines Helixbogens erstreckenden Strahlenquelle erfassen würde, die zur Detektorebene senkrechte und zur Rotationsachse parallele Strahlenfächer emittiert. Alle Meßwerte, die zu parallelen Strahlenfächern gehören, bilden dabei eine Gruppe, die sich aus einer Anzahl von Untergruppen zusammensetzt. Jede Untergruppe umfaßt die Meßdaten, die einer gemeinsamen Filteroperation unterzogen werden und die einer horizontalen Zeile des (virtuellen) Detektors zugeordnet sind. Trotz einer ansprechenden Qualität des durch eine Rückprojektion aus den gefilterten Meßdaten erzeugten CT-Bildes können sich auch darin noch Bildartefakte zeigen, insbesondere wenn das kegelförmige Strahlenbündel senkrecht zur Rotationsachse und parallel dazu einen großen Öffnungswinkel hat.

Es ist Aufgabe der vorliegenden Erfindung, die Bildqualität bei einem Verfahren der eingangs genannten Art noch weiter zu verbessern. Diese Aufgabe wird gelöst durch eine solche Unterteilung in Untergruppen, dass die Messdaten verschiedener Untergruppen aus unterschiedlichen Projektionen von Voxeln aus zumindest näherungsweise derselben Fläche innerhalb des Untersuchungsbereiches resultieren, wobei ein Rebinning zu Gruppen von Messdaten erfolgt, die Strahlen zugeordnet sind, die in zueinander und zur Rotationsachse parallelen Ebenen liegen.

Jede Gruppe von Messdaten umfasst die Projektion sämtlicher jeweils im Strahlengang befindlicher Voxel in einer bestimmten Projektionsrichtung. Zu jeder Untergruppe von Messdaten, die einer (gemeinsamen) Filteroperation unterzogen werden, gehört ein Teil dieser Voxel. Bei dem bekannten Verfahren ändert sich von Projektion zu Projektion die Zusammensetzung der Voxel, zu denen die einer gemeinsamen Filteroperation unterzogenen Messdaten gehören. Die Erfindung basiert auf der Erkenntnis, dass die Bildartefakte des bekannten Verfahren aus dieser sich ständig ändernden Zusammensetzung resultieren. Die Erfindung sieht daher vor, dass die Filteroperationen in den unterschiedlichen Gruppen von Messdaten bzw. für die unterschiedlichen Projektionen stets diejenigen Messdaten erfassen, die - zumindest näherungsweise - aus der Projektion derselben Voxel in dem Untersuchungsobjekt resultieren. Dadurch ergibt sich eine Verbesserung der Bildqualität.

Der Erfindung liegt das Prinzip zugrunde, die Filteroperation jeweils so durchzuführen, dass sie die durch unterschiedlichen Projektionen jeweils der gleichen Voxel resultierenden Messdaten erfassen..

Wird die in Anspruch 1 definierte Fläche aus einer anderen Richtung projiziert, dann beschreibt die Projektion auf der Detektoreinheit in der Regel keine Linie mehr, sondern eine - langgestreckte - Fläche. Obwohl gemäß der Weiterbildung nach Anspruch 2 nur eine eindimensionale Filterung entlang einer (gekrümmten) Linie erfolgt, die diese Fläche approximiert, werden Bildartefakte weitgehend unterdrückt.

Demgegenüber erfolgt bei Anspruch 3 eine eindimensionale Filteroperation entlang einer die Fläche approximierenden Geraden, wobei sich ebenfalls eine Verbesserung der Bildqualität ergibt.

Bei der bevorzugten Ausgestaltung nach Anspruch 4 beschreiben die Strahlenquelle und die Detektoreinheit um jedes Voxel von seinem Eintritt in das kegelförmige Strahlenbündel bis zu seinem Austritt einen Winkel von genau 180 DEG (von dem jeweiligen Voxel ausgesehen).

Die beste erreichbare Bildqualität ergibt sich, wenn bei der Rekonstruktion eine Rückprojektion erfolgt, bei der die rückprojizierten Strahlen im dreidimensionalen Raum entlang derselben Pfade verlaufen, entlang denen die Messwerte erfasst wurden. Bei der Ausgestaltung nach Anspruch 5 erfolgt demgegenüber zwecks Vereinfachung eine Rückprojektion in einer ebenen Schicht, die die Fläche approximiert, in der die einer gemeinsamen Filteroperation unterzogenen Voxel liegen. Die Bildqualität ist jedoch nicht so gut wie das aus einer dreidimensionalen Rekonstruktion abgeleitete Bild der betreffenden Schicht. Das Verfahren nach Anspruch 5 kann auch bei mehreren, bezüglich der Rotationsachse nutierenden (zweidimensionalen) Schichten wiederholt werden, aus denen sich ein dreidimensionaler Bereich (mit geringerer Bildqualität) rekonstruieren lässt. Dies ist an sich aus der WO 98/448847 bekannt.

Ein Computertomograph zur Durchführung des erfindungsgemäßen Verfahrens ist in Anspruch 6 beschrieben.

Die Erfindung wird nachstehend anhand der Zeichnungen näher erläutert. Es zeigen:

Fig. 1    einen Computertomographen, mit dem das erfindungsgemäße Verfahren durchführbar ist,
Fig. 2    ein Ablaufdiagramm mit den einzelnen Schritten des Verfahrens,
Fig. 3    die dem Rebinning zugrunde gelegten geometrischen Verhältnisse,
Fig. 4    verschiedene Projektionen derselben Voxel und

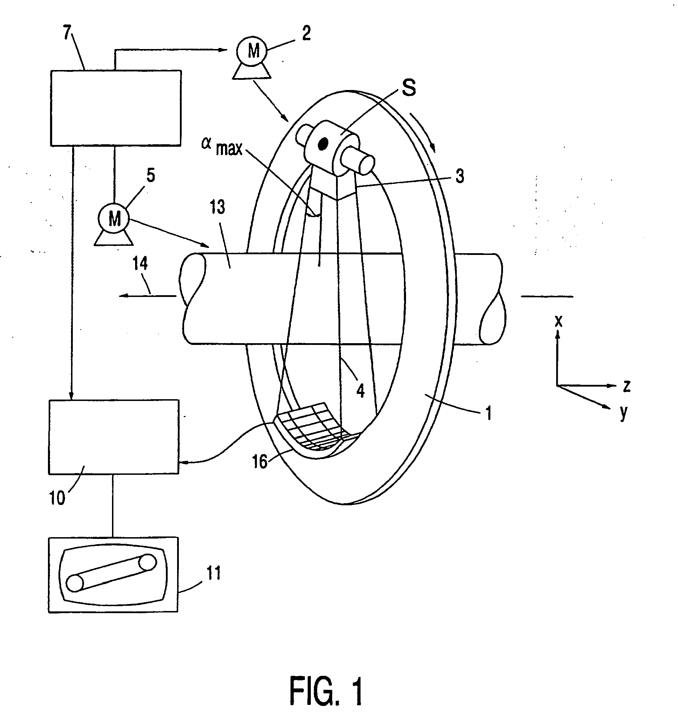

Der in Fig. 1 dargestellte Computertomograph umfaßt eine Gantry 1, die um eine parallel zur z-Richtung verlaufende Rotationsachse 14 rotieren kann. Dazu wird die Gantry 1 von einem Motor 2 mit einer vorzugsweise konstanten, aber einstellbaren Winkelgeschwindigkeit angetrieben. An der Gantry ist eine Strahlenquelle S, beispielsweise ein Röntgenstrahler, befestigt. Dieser ist mit einer Kollimatoranordnung 3 versehen, die aus der von der Strahlenquelle S erzeugten Strahlung ein kegelförmiges Strahlenbündel 4 ausblendet, d.h. ein Strahlenbündel, das sowohl in Richtung der z-Achse als auch in einer dazu senkrechten Richtung, d.h. in der x-y-Ebene des in Fig. 1 dargestellten Koordinatensystems, eine von Null verschiedene endliche Ausdehnung hat.

Das Strahlenbündel 4 durchdringt ein nicht näher dargestelltes Objekt, das sich in einem Untersuchungsbereich 13 befindet. Der Untersuchungsbereich 13 hat die Form eines Zylinders, der im folgenden auch als Objektzylinder bezeichnet wird. Nach dem Durchsetzen des Objektzylinders trifft das Röntgenstrahlenbündel 4 auf eine an der Gantry 1 befestigte zweidimensionale Detektoreinheit 16, die eine Anzahl von Detektorzeilen mit jeweils einer Vielzahl von Detektorelementen umfaßt. Jedes Detektorelement erfaßt in jeder Strahlenquellenposition einen Strahl aus dem Strahlenbündel 4 und liefert einen der Intensität dieses Strahles entsprechenden Meßwert. Die Detektoreinheit 16 kann auf einem Kreisbogen um die Rotationsachse 14 angeordnet sein, jedoch sind auch andere Detektorgeometrien möglich, z.B. die Anordnung auf einem Kreisbogen um die Strahlenquelle S.

Der mit $\alpha_{max}$ bezeichnete Öffnungswinkel des Strahlenbündels 4 (als Öffnungswinkel ist der Winkel definiert, den ein in der x-y-Ebene am Rande liegender Strahl des Bündels 4 mit einer durch die Strahlenquelle S und die Rotationsachse 14 definierten Ebene einschließt und bestimmt dabei den Durchmesser des Objektzylinders 13, innerhalb dessen das zu untersuchende Objekt sich bei der Akquisition der Meßwerte befinden muß. Der Untersuchungsbereich 13 bzw. ein darin befindliches Objekt - beispielsweise ein auf einem Patientenlagerungstisch befindlicher Patient - kann mittels eines Motors 5 parallel zur Richtung der Rotationsachse 14 bzw. der z-Achse verschoben werden. Die Geschwindigkeit dieses Vorschubs in z-Richtung ist vorzugsweise konstant und einstellbar.

Die von der Detektoreinheit 16 akquirierten Meßdaten werden einem Bildverarbeitungsrechner 10 zugeführt, der daraus die Absorptionsverteilung in dem vom Strahlenkegel 4 erfaßten Teil des Untersuchungsbereichs 13 rekonstruiert und z.B. auf einem Monitor 11 wiedergibt. Die beiden Motoren 2 und 5, der Bildverarbeitungsrechner 10, die Strahlenquelle S und der Transfer der Meßdaten von der Detektoreinheit 16 zum Bildverarbeitungsrechner 10 werden von einer geeigneten Kontrolleinheit 7 gesteuert.

Wenn der Motor 5 still steht und der Motor 2 die Gantry 1 rotieren läßt, ergibt sich eine kreisförmige Abtastbewegung der Strahlenquelle S und der Detektoreinheit 16. Die Kontrolleinheit kann die Motoren 2 und 5 aber auch so steuern, dass das Verhältnis der Vorschubgeschwindigkeit des Untersuchungsbereichs 13 und der Winkelgeschwindigkeit der Gantry konstant ist. In diesem Fall bewegen sich die Strahlenquelle S und ein im Untersuchungsbereich 13 befindliches Objekt relativ zueinander auf einer helixförmigen Bahn. Im folgenden soll nur diese helixförmige Abtastbewegung betrachtet werden. Im Prinzip ist es dabei gleichgültig, ob die Abtasteinheit 16 bzw. der Untersuchungsbereich 13 die Rotations- bzw. Vorschubbewegung ausführen; wesentlich ist allein die Relativbewegung.

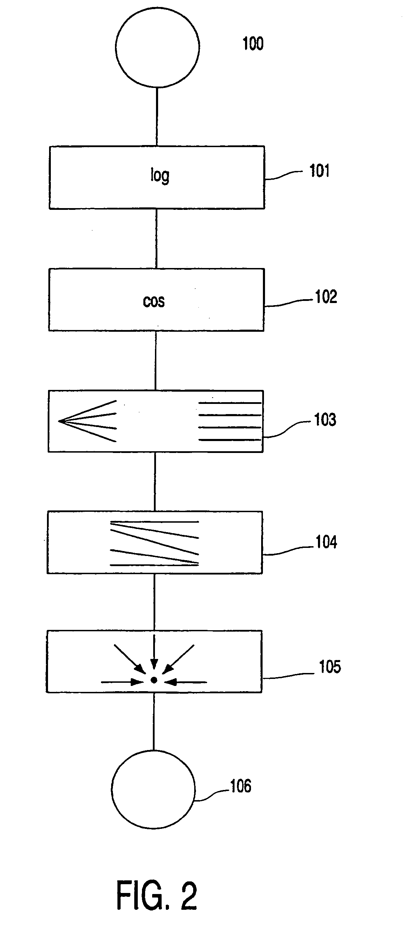

Im folgenden wird unter Bezugnahme auf das in Fig. 2 dargestellte Ablaufdiagramm erläutert, wie die von den Detektorelementen der Detektoreinheit 16 während einer Untersuchung gelieferten Signale in dem Bildverarbeitungsrechner 10 weiter verarbeitet werden. Nach der Initialisierung (Block 100) werden die Signale der Detektorelemente akquiriert (Schritt 101). Für die nachfolgende Rekonstruktion der Absorptionsverteilung werden Signale nur von denjenigen Detektorelementen herangezogen, die sich innerhalb eines Detektorfensters befinden (als Detektorfenster wird hierbei und im folgenden der Teil der Meßfläche bezeichnet, der die für die Rekonstruktion erforderlichen Daten - und nur diese - erfaßt). Dieses Detektorfenster ist in Richtung der z-Achse durch die Projektion zweier aufeinander folgender Windungen der Helix definiert. Es läßt sich zeigen, dass bei einer solchen Gestaltung des Detektorfensters die Strahlenquelle jedes Voxel im Untersuchungsbereich bei seinem Eintritt und bei seinem Austritt aus dem Strahlenbündel aus um exakt 180° gegeneinander versetzten Positionen (bezogen auf das jeweilige Voxel) auf das Detektorfenster projiziert.

Die Beschränkung auf Signale innerhalb dieses Detektorfensteres kann dadurch erreicht werden, dass die Detektoreinheit entsprechend geformt ist. Wenn die Detektoreinheit die Form eines Kreisbogens um die Rotationsachse hätte, müßte die Abwicklung des Detektorfensters die Form eines Parallelogramms haben; wenn die Detektoreinheit (in einer zur Rotationsachse senkrechten Ebene) einen Kreisbogen um die Strahlenquelle definiert, ergäbe sich ein verzerrtes Parallelogramm. Detektoreinheiten mit solchen Formen der Abwicklung sind aufwendig zu realisieren und für Messungen ungeeignet, bei denen die Relativbewegung zwischen Strahlenquelle und Untersuchungsobjekt nicht helixformig, sondern kreisförmig ist. Man kann aber auch eine Detektoreinheit verwenden, deren Abwicklung die Form eines Rechtecks hat, das so groß ist, dass es die Abwicklung des Detektorfensters umschließt. Man kann dabei durch geeignet geformte Kollimatoren 3 erreichen, dass die Röntgenstrahlung nur Detektorelemente innerhalb des Detektorfensters trifft. Statt dessen wäre es auch möglich, die gesamte (in der Abwicklung rechteckige) Meßfläche zu bestrahlen und die Signale von Detektorelementen außerhalb des Detektorfensters bei der Rekonstruktion unberücksichtigt zu lassen. Wenn das Untersuchungsobjekt ein Patient wäre, würde dieser dadurch allerdings unnötig einer erhöhten Strahlenbelastung ausgesetzt.

Die Signale der Detektorelemente werden zunächst digitalisiert und durch einen Referenzwert dividiert, und der daraus resultierende Quotient wird logarithmiert. Die auf diese Weise entstehenden Meßwerte stellen das Linienintegral der Absorption der Strahlung längs eines die Strahlenquelle mit dem jeweiligen Detektorelement verbindenden Strahls dar. Es ist dann Aufgabe der nachfolgenden Verarbeitungsschritte, aus diesen Linienintegralen der Absorption die räumliche Verteilung der Absorption zu bestimmen. Im Schritt 102 werden die Meßwerte mit einem Faktor multipliziert, der dem Cosinus des Winkels der zu den Meßwerten gehörenden Strahlen mit einer zur Rotationsachse 14 senkrechten Ebene entspricht. Dieser Schritt kann aber in den Fällen entfallen, in denen der Abstand zweier Helixwindungen klein im Vergleich zu deren Radius ist. In diesen Fällen ist der genannte Winkel klein, so dass der Cosinus dieses Winkels stets praktisch den Wert 1 hat. Es ist auch möglich, die Reihenfolge dieses Schrittes und des nachfolgenden Schrittes zu vertauschen.

Im Schritt 103 erfolgt ein Rebinning, d.h. ein Verarbeitungsschritt, der das Umsortieren und Interpolieren der Meßwerte in Gruppen umfaßt, die für den nachfolgenden Filterungsschritt (Block 104) besonders geeignet sind. Ausgangspunkt sind dabei die Meßwerte, die von der realen, aus Fig. 1 ersichtlichen Anordnung von Strahlenquelle S mit kegelförmigem Strahlenbündel 4 und dem zweidimensionalen Detektor 16 erzeugt werden. Jeder der für die Rekonstruktion herangezogenen Meßwerte ist dabei durch die Position der Strahlenquelle auf der helixförmigen Bahn 17 (der die Annahme zugrundeliegt, dass das Untersuchungsobjekt ruht, während die Strahlenquelle und der Detektor die helixförmige Relativbewegung ausführen) definiert, sowie durch die Lage des Detektorelements innerhalb der Detektoreinheit 16, das diesen Meßwert erfaßt hat.

Das im Schritt 103 erfolgte Rebinning liefert somit Gruppen von Meßdaten, die sich ergeben würden, wenn ein ebener rechteckiger Detektor in einer die Rotationsachse 14 enthaltenden Ebene die Meßdaten einer sich entlang eines helixförmigen Bogens 17 erstreckenden Strahlenquelle erfassen würde, die zur Detektorebene senkrechte und zur Rotationsachse 14 parallele Strahlenfächer emittiert.

Das kegelförmige Strahlenbündel 4 kann man sich nämlich als aus einer Anzahl von Strahlenfächern zusammengesetzt vorstellen, die von der jeweiligen Strahlenquellenposition ausgehen und die in zur Rotationsachse parallelen Ebenen liegen. Für andere Positionen ergeben sich entsprechende Sätze von Strahlenfächern. Die in zueinander parallelen Ebenen befindlichen Strahlenfächer - und die zu diesen Strahlenfächern gehörenden Meßwerte - können in einer Gruppe zusammengefaßt werden. Diese Gruppe enthält somit alle Meßwerte, die die Absorption des Untersuchungsbereichs für eine bestimmte Projektionsrichtung definieren (als Projektionsrichtung wird die Richtung der auf eine zur Rotationsachse senkrechte (x-y-)Ebene projizierten Strahlen bezeichnet).

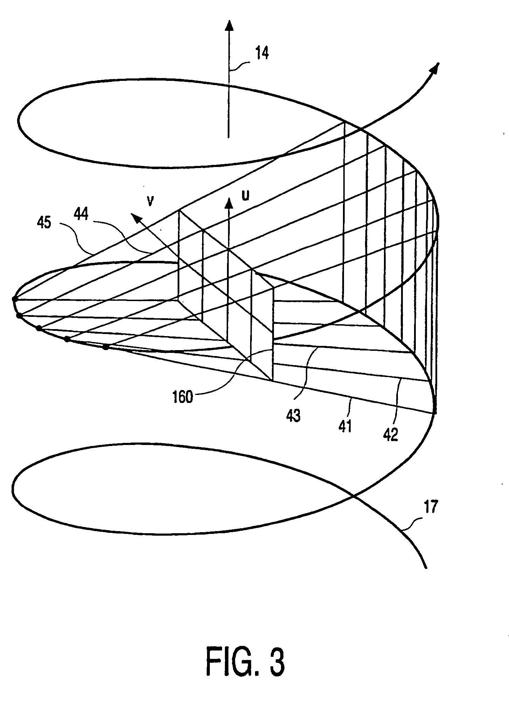

Dies ist in Fig. 3 dargestellt, die die zu einer Gruppe von Meßwerten gehörenden Strahlenfächer 41....45 darstellt. Diese Strahlenfächer liegen in parallelen Ebenen. Alle Punkte eines solchen Strahlenfächers gehen von der helixförmigen Bahn 17 aus. Der obere Randstrahl dieser Strahlenbündel verläuft durch einen gegenüberliegenden Bogen der Helix 17 und alle unteren Randstrahlen der Strahlenfächer verlaufen durch einen Helixbogen, der gegenüber dem ersten Bogen um eine Windung der Helix nach unten versetzt ist.

Es läßt sich zeigen, dass der obere und untere Rand aller zu einander paralleler Strahlenfächer eine zur Projektionsrichtung senkrechte und die Rotationsachse 14 enthaltenden Ebene entlang zweier paralleler horizontaler Geraden durchstoßen. Die beiden Geraden definieren in Verbindung mit der erwähnten Ebene einen virtuellen Detektor 160, und ihr Abstand Geraden voneinander entspricht der Hälfte des Abstandes zweier benachbarter Windungen der Helix. Das Rebinning liefert für alle Projektionsrichtungen und für alle Punkte eines äquidistanten Gitters mit den Koordinaten u,v auf dem virtuellen Detektor 160 Meßdaten - ggf. durch Interpolation.

Insoweit als bisher beschrieben, ist das Verfahren aus der eingangs genannten Veröffentlichung bekannt.

Bei dem bekannten Verfahren schließt sich nun ein Filterschritt an, der die eindimensionale Filterung derjenigen Meßdaten umfaßt, die einer (in v-Richtung verlaufenden) horizontalen Zeile virtuellen Detektors zugeordnet sind. Der zweidimensionale Satz von Voxeln, die bei dieser eindimensionalen Filteroperation für eine bestimmte Projektionsrichtung zusammenwirken, wirkt für andere Projektionsrichtungen nur noch teilweise oder gar nicht mehr zusammmen.

Die Erfindung beruht auf der Erkenntnis, dass sich demgegenüber eine Bildverbesserung ergibt, wenn jede der Filteroperationen im Schritt 104 jeweils die Meßdaten erfaßt, die für die verschiedenen Projektionsrichtungen jeweils aus der Projektion derselben Voxel resultieren.

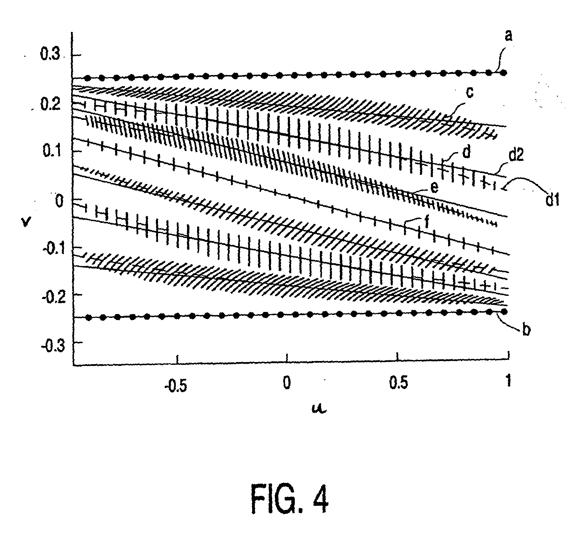

Zur Erläuterung wird auf Fig. 4 verwiesen. Fig. 4 stellt die Projektion der Voxel, die sich im Untersuchungsbereich auf den oberen Randstrahlen des in Fig. 3 dargestellten Strahlenbündels befinden, für jeweils um 22,5° gegeneinander versetzte Projektionsrichtungen dar. Definitionsgemäß werden diese Voxel bei ihrem Eintritt in die Strahlenfächer 41... 45 auf eine horizontale gerade Linie a projiziert, die den oberen Rand des Detektors bildet. Nach einer Änderung der Projektionsrichtung um 180° (wobei sich die Fächer um eine halbe Windung der Helix nach oben bewegen und der virtuelle Detektor 160 mit ihr) werden dieselben Voxel im Untersuchungsbereich ebenfalls auf eine horizontale Gerade (b) projiziert, nämlich den unteren Rand des virtuellen Detektors 160. Für die dazwischen liegenden Projektionsrichtungen werden die gleichen Voxel nicht mehr auf eine Gerade projiziert, sondern auf schmale Streifen - c, d, e, f, usw.-, die gegenüber den horizontalen Geraden a und b mehr oder weniger geneigt sind.

Diese Streifen überlappen einander wenn man eine größere Zahl von Projektionsrichtungen in Betracht zieht. Streng genommen ist es also gar nicht möglich, dass die Filteroperationen für die verschiedenen Projektionsrichtungen jeweils die Meßdaten erfassen, die jeweils aus der Projektion exakt derselben Voxel resultieren. Zumindest näherungsweise läßt sich dies aber dadurch erreichen, dass die Filterung längs einer Linie erfolgt, die den Streifen approximiert, auf den die Voxel projiziert werden. Diese Linie kann gekrümmt sein wie die gestrichelte Linie d1, oder aber eine Gerade, wie die in ausgezogenen Strichen dargestellte Gerade d2.

Eine solche Gerade ergibt sich als Schnittgerade einer Ebene, die die durch die oberen Randstrahlen der Strahlenfächer 41...45 definierte Fläche approximiert. Diese Ebene ist durch den oberen Randstrahl des durch die Rotationsachse 14 verlaufenden Strahlenfächers und den oberen Rand des virtuellen Detektors in der in Fig. 3 dargestellten Position definiert. Die Gleichung dieser Geraden lautet

$$u = m - v\frac{P}{4R\sqrt{1 + (1 + \frac{P}{4R})^2)\tan^2(m\frac{2\pi}{P})}} \qquad (1)$$

R ist dabei der Radius des durch die Projektion der Helix 17 auf eine zur Rotationsachse senkrechte Ebene entstehenden Kreises. P ist der Abstand zweier benachbarter Windungen der Helix, m ist ein Parameter, der zwischen -P/4 und P/4 definiert ist, u ist eine vom Zentrum des virtuellen Detektors in Richtung der Rotationsachse verlaufende Koordinate, und v ist eine dazu senkrechte Koordinate auf dem virtuellen Detektor, wie aus Fig. 3 ersichtlich.

Die Gleichung 1 (und ebenso die Darstellung der verschiedenen Projektionen derselben Fläche auf den Detektor 160 gemäß Fig.4) gilt nur für die aus Fig. 3 ersichtliche helixförmige Bahn, auf der die Strahlenquelle, die Rotationsachse in Gegenuhrzeigersinn umläuft, wenn sie sich nach oben bewegt. Würde hingegen die Rotationsachse dabei von der Strahlenquelle im Uhrzeigersinn umlaufen, dann müßten die Werte u in Gleichung (1) mit dem Faktor -1 multipliziert werden (und die Streifen in Fig. 4 würden von links oben nach rechts unten verlaufen).

Die Filterung entlang der geraden oder gekrümmten Linie kann im Prinzip dadurch erfolgen, dass die sich aus dem Rebinning entlang dieser Linien ergebenden Daten einer Faltung mit einem geeigneten eindimensionalen Faltungskern unterzogen werden. Eine einfachere Möglichkeit besteht darin, die durch das Rebinning entstandenen Meßdaten zunächst einer Fouriertransformation zu unterziehen. Die auf diese Weise in den Ortsfrequenzraum transformierten Daten werden einer rampenförmigen Filterung entlang der Linie unterzogen, wobei die Dämpfung linear mit zunehmendem Betrag der Frequenz abnimmt. Die auf diese Weise im Ortsfrequenzraum gefilterten Daten werden einer inversen Fouriertransforrnation unterzogen, so dass sie gefilterte Meßdaten ergeben.

Im nächsten Schritt 105 erfolgt eine Rückprojektion anhand der gefilterten Meßdaten. Die Meßdaten werden dabei entlang der gleichen (durch das Rebinning ggf. geringfügig modifizierten) Strahlenpfade in den Untersuchungsbereich rückprojiziert, längs derer sie akquiriert wurden. Die Absorptionswerte für ein einzelnes Voxel des Untersuchungsbereichs ergeben sich aus der Überlagerung sämtlicher (gefilterter) Meßdaten, die bei der Akquisition von der Projektion dieses Voxels beeinflußt wurden. Für jedes Voxel ergeben sich dabei Beiträge von Strahlen aus einem Winkelbereich von exakt 180°.

**Patentansprüche**

1. Computertomographie-Verfahren mit den Schritten

- Erzeugen eines kegelförmigen, einen Untersuchungsbereich (13) bzw. ein darin befindliches Objekt durchsetzenden Strahlenbündels (4) mit einer Strahlenquelle (S),
- Erzeugung einer eine Rotation um eine Rotationsachse (14) und eine Verschiebung parallel zur Rotationsachse (14) umfassenden Relativbewegung zwischen der Strahlenquelle (S) einerseits und dem Untersuchungsbereich (13) bzw. dem Objekt anderseits,

- Akquisition von Messwerten, die von der Intensität in dem Strahlenbündel (4) jenseits des Untersuchungsbereiches (13) abhängen, mit einer Detektoreinheit (16) während der Relativbewegung,

- Rebinning der Messwerte zu einer Anzahl von Gruppen von Messdaten, sodass jede Gruppe nur solche Messdaten umfaßt, die in zueinander und zur Rotationsachse (14) parallelen Ebenen liegenden Strahlen (41-45) zugeordnet sind,

- Filterung der durch das Rebinning erzeugten Messdaten der Gruppen, die Filteroperationen an allen Untergruppen von Messdaten umfaßt, wobei eine solche Unterteilung in Untergruppen erfolgt, dass die Messdaten jeder Untergruppe aus unterschiedlichen Projektionen von Voxeln aus zumindest näherungsweise derselben Fläche innerhalb des Untersuchungsbereiches (13) resultieren und aus der Projektion von Voxeln resultieren, die - zumindest näherungsweise - gleichzeitig in das Bündel von Strahlen (41-45) eintreten bzw. daraus austreten,

- Rekonstruktion der Absorption in Voxeln des Untersuchungsbereiches (13) durch Rückprojektion der gefilterten Messdaten aus verschiedenen Gruppen.

**2.** Computertomographie-Verfahren nach Anspruch 1,
**dadurch gekennzeichnet,**
**dass** die Filteroperationen eine eindimensionale Filterung derjenigen Messdaten umfassen, deren zugehörige Strahlen eine die Projektion der Fläche auf die virtuelle Detektoreinheit (160) approximierende gekrümmte Linie durchstoßen.

**3.** Computertomographie-Verfahren nach Anspruch 1,
**dadurch gekennzeichnet,**
**dass** jede Filteroperation eine eindimensionale Filterung derjenigenMessdaten umfasst, deren zugehörige Strahlen eine die Projektion der Fläche auf die virtuelle Detektoreinheit (160) approximierende gerade Linie durchstoßen.

**4.** Computertomographie-Verfahren nach Anspruch 1,
**dadurch gekennzeichnet,**
**dass** Messdaten nur von solchen Strahlen herangezogen werden, die in dem Bereich zwischen zwei in Richtung der Rotationsachse benachbarten Bögen der Helix (17) verlaufen.

**5.** Computertomographie-Verfahren nach Anspruch 3,
**dadurch gekennzeichnet,**
**dass** die Fläche durch eine Ebene approximiert wird, dass die einzelnen Untergruppen, über die eine Filteroperation erfolgt, durch die Messdaten definiert sind die entlang einer in der Ebene befindlichen Geraden gemessen wurden und dass die gefilterten Messdaten zur zweidimensionalen Rekonstruktion der Absorption in den Voxeln der Ebene herangezogen werden.

**6.** Computertomograph Zur Durchführung des Verfahrens nach Anspruchs 1 mit einer Strahlenquelle (S) die ein kegelförmiges Strahlenbündel (4) erzeugt und einer damit gekoppelten Detektoreinheit(16) sowie mit einer Antriebsanordnung(2, 5), um ein im Untersuchungsbereich (13) enthaltenes Objekt und die Strahlenquelle (S) relativ zueinander eine helixförmige Relativbewegung ausführen zu lassen und mit einer Rekonstruktionseinheit(10) zur Rekonstruktion der räumlichen Verteilung der Absorption innerhalb des Untersuchungsbereiches (13) aus den von der Detektoreinheit (16) akquirierten Messdaten,
wobei der Computertomograph aufweist:

Mittel zum Rebinning der Messdaten in einer Anzahl von Gruppen von Messdaten, wobei jede Gruppe nur solche Messdaten umfaßt, die in zueinander und zur Rotationsachse (14) parallelen Ebenen liegenden Strahlen (41-45) zugeordnet sind, und

Mittel zur Filterung der durch das Rebinning erzeugten Messdaten einer jeden Gruppe, die Filteroperationen an allen Untergruppen von Messdaten umfasst, wobei eine Unterteilung der Gruppen in Untergruppen erfolgt, und wobei die Messdaten jeder Untergruppe aus der Projektion von Voxeln aus zumindest näherungsweise derselben Fläche innerhalb des Untersuchungsbereiches (13) resultierten und aus der Projektion von Voxeln resultieren, die - zumindest näherungsweisegleichzeitig in das Bündel von Strahlen (41-45) eintreten bzw. daraus austreten.

**Claims**

1. A computer tomography method which includes the following steps:

   - generating by a radiation source (S) a conical radiation beam (4) traversing an examination zone (13) or an object situated therein,
   - generating a relative motion comprising a rotation about an axis of rotation (14) and a displacement parallel to the axis of rotation (14), between the radiation source (S), on the one hand, and the examination zone (13) or the object, on the other,
   - acquiring, during the relative motion and using a detector unit (16), measured values which are dependent on the intensity in the radiation beam (4) on the other side of the examination zone (13),
   - rebinning the measured values so as to form a number of groups of measured data, so that each group contains only those measured data that are assigned to fan beams (41 - 45) situated in planes parallel to each other and to the axis of rotation (14)
   - filtering the measured data of the groups formed by the rebinning, which filtering involves filtering operations performed on all the sub-groups of measured data,

   **characterized in that** the subdivision into sub-groups is such that the measured data of each sub-group result from different projections of voxels from at least approximately the same surface within the examination zone (13) and result from the projection of voxels which at least approximately simultaneously enter the fan beam (41 - 45) or emerge therefrom,

   - reconstructing the absorption in voxels of the examination zone by back projection of the filtered measured data of different groups.

2. A computer tomography method as claimed in claim 1,
   **characterized in that** the filtering operations comprise one dimensional filtering of those measured data whose associated rays intersect a curved line approximating the projection of the surface of the virtual detector unit (160).

3. A computer tomography method as claimed in claim 1,
   **characterized in that** each filtering operation comprises a one-dimensional filtering of those measured data whose associated rays intersect a straight line approximating the projection of the surface on the virtual detector unit (160).

4. A computer tomography method as claimed in claim 1,
   **characterized in that** use is made exclusively of measured data from rays which extend in the zone between two arcs of the helix (17) which neighbor one another in the direction of the axis of rotation.

5. A computer tomography method as claimed in claim 3,
   **characterized in that** the surface is approximated by a plane, that the individual sub-groups via which a filtering operation is performed are defined by the measured data measured along a straight line present in the plane, and that the filtered measured data are used for the two-dimensional reconstruction of the absorption in the voxels of the plane.

6. A computer tomograph for carrying out the method as claimed in claim 1, comprising a radiation source (S) generating a conical radiation beam (4) and a detector unit (16) which is coupled thereto as well as a drive arrangement (2, 5) for making an object present in the examination zone (13) and the radiation source (S) perform a helical relative motion relative to one another, and also comprising a reconstruction unit (10) for the reconstruction of the spatial distribution of the absorption within the examination zone (13) from the measured data acquired by the detector unit (16),
   **characterized in that** the computer tomograph comprises:

   means for rebinning the measured data into a number of groups of measured data, - where each group contains only those measured data that are assigned to fan beams (41 - 45) situated in planes parallel to each other and to the axis of rotation (14), and
   - means for filtering the measured data of each group formed by the rebinning, which filtering involves filtering operations performed on all the sub-groups of measured data, where the groups are divided into sub-groups, the measured data of each sub-group result from the projection of voxels from at least approximately the same surface within the examination zone (13) and result from the projection of voxels which, at least approximately,

simultaneously enter the fan of beams (41 - 45) or emerge therefrom.

**Revendications**

1. Procédé de tomographie informatisée comportant les étapes suivantes :

   - production d'un faisceau de rayons (4) de forme conique traversant une zone d'examen (13) ou un objet qui s'y trouve avec une source de rayons (S),
   - production d'un mouvement relatif comprenant une rotation autour d'un axe de rotation (14) et un décalage parallèlement à l'axe de rotation (14) entre la source de rayons (S), d'une part, et la zone d'examen (13) ou l'objet, d'autre part,
   - acquisition de valeurs de mesure qui dépendent de l'intensité dans le faisceau de rayons (4) au-delà de la zone d'examen (13), avec une unité détectrice (16) pendant le mouvement relatif,
   - rebinning des valeurs de mesure en plusieurs groupes de données de mesure de telle sorte que chaque groupe comprenne seulement des données de mesure qui sont attribuées à des rayons (41-45) se trouvant dans des plans parallèles l'un par rapport à l'autre et par rapport à l'axe de rotation (14),
   - filtrage des données de mesure des groupes, produites par le rebinning, qui comprend des opérations de filtrage à tous les sous-groupes de données de mesure, la répartition en sous-groupes étant effectuée de telle sorte que les données de mesure de chaque sous-groupe résultent de différentes projections de voxels à partir d'une surface identique, du moins approximativement, dans la zone d'examen (13) ou résultent de la projection de voxels qui entrent simultanément - du moins approximativement - dans le faisceau de rayons (41-45) ou en sortent,
   - reconstruction de l'absorption dans des voxels de la zone d'examen (13) par rétroprojection des données de mesure filtrées à partir de différents groupes.

2. Procédé de tomographie informatisée selon la revendication 1,
   **caractérisé en ce**
   **que** les opérations de filtrage comprennent un filtrage unidimensionnel des données de mesure dont les rayons correspondants traversent une ligne courbe approchant la projection de la surface sur l'unité de détecteur virtuelle (160).

3. Procédé de tomographie informatisée selon la revendication 1,
   **caractérisé en ce**
   **que** chaque opération de filtrage comprend un filtrage unidimensionnel des données de mesure dont les rayons correspondants traversent une ligne droite approchant la projection de la surface sur l'unité détectrice virtuelle (160).

4. Procédé de tomographie informatisée selon la revendication 1,
   **caractérisé en ce**
   **que** les données de mesure sont utilisées uniquement par les rayons qui s'étendent dans la zone entre deux arcs de l'hélice (17) voisins dans la direction de l'axe de rotation.

5. Procédé de tomographie informatisée selon la revendication 3,
   **caractérisé en ce**
   **que** la surface est approchée par un plan, que les différents sous-groupes par l'intermédiaire desquels une opération de filtrage est effectuée sont définis par les données de mesure qui ont été mesurées le long d'une droite se trouvant dans le plan et que les données de mesure filtrées sont utilisées pour la reconstruction bidimensionnelle de l'absorption dans les voxels du plan.

6. Tomographe informatisé pour l'exécution du procédé selon la revendication 1, avec une source de rayons (S) qui produit un faisceau de rayons de forme conique (4) et une unité de détecteur (16) couplée à celle-ci ainsi qu'avec un dispositif d'entraînement (2, 5) pour faire exécuter un mouvement relatif hélicoïdal par un objet contenu dans la zone d'examen (13) et la source de rayons (S) l'un par rapport à l'autre et avec une unité de reconstruction (10) pour la reconstruction de la répartition spatiale de l'absorption dans la zone d'examen (13) à partir des données de mesures acquises par l'unité de détecteur (16), le tomographe informatisé présentant :

   - des moyens de rebinning des données de mesure dans plusieurs groupes de données de mesure, chaque groupe comprenant seulement les données de mesure qui sont affectées à des rayons (41 - 45) se trouvant dans des plans parallèles l'un à l'autre et à l'axe de rotation (14) et

des moyens de filtrage des données de mesure de chaque groupe, produites par le rebinning, qui comprennent des opérations de filtrage à tous les sous-groupes de données de mesure, la répartition des groupes en sous-groupes étant effectuée de telle sorte que les données de mesure de chaque sous-groupe résultent de différentes projections de voxels à partir d'une surface identique, du moins approximativement, dans la zone d'examen (13) ou résultent de la projection de voxels qui entrent simultanément - du moins approximativement - dans le faisceau de rayons (41-45) ou en sortent.

**FIG. 1**

FIG. 2

FIG. 3

FIG. 4